# EUROPEAN PATENT APPLICATION

(11) **EP 0 859 059 A2**
(43) Date of publication of application: **19.08.1998**
(21) Application number: 97304546.1
(22) Date of filing: 26.06.1997
(51) Int. Cl.: C12N 15/90, A01K 67/027, C12N 15/55, C12N 9/16

(54) **Methods and materials for introducing exogenous genes into cultured cells or fertilized eggs**

(30) Priority: 17.01.1997 JP 6550/97
(71) Applicant: DIRECTOR-GENERAL, NATIONAL INSTITUTE OF ANIMAL INDUSTRY, Inashiki-gun, Ibaragi-ken (JP); President, Hiroshima University, Higashihiroshima-shi, Hiroshima-ken (JP)
(72) Inventor: Yasue, Hiroshi, Tsukuba-shi, Ibaraki-ken, 305 (JP); Shimada, Hiraku, Naka-ku, Hiroshima 730 (JP); Akasaka, Koji, Higashihiroshima-shi, Hiroshima, 739-01 (JP)
(74) Representative: BATCHELLOR, KIRK & CO.

(57) **Abstract**

There is provided a method of introducing exogenous genes into cultured cells or fertilized eggs, characterized in that each exogenous gene is introduced into chromosome DNA in a cultured cell or fertilized egg under a condition where the exogenous gene is placed between insulators so as to insure an insulated environment, such that the introduced exogenous gene will not be affected by any influence from adjacent genes in the cultured cell or fertilized egg.

## Description

### BACKGROUND OF THE INVENTION

The present invention relates to a gene introduction technique for use in various scientific researches, such as biological research, medical research, livestock research, fishery research. In particular, the present invention relates to methods and materials for introducing exogenous genes into cultured cells or fertilized eggs.

Recently, with the development of exogenous gene introduction techniques, it has become possible to produce new biological varieties having new and useful forms/properties, thereby providing transgenic organisms. This is considered to be an extremely significant achievement never attained before.

Up till now, various methods have been used as exogenous gene introduction techniques to introduce exogenous genes (cloned on plasmid vectors) into cultured cells or fertilized eggs. Such methods include, for example, the calcium phosphate method, the microinjection method, the virus vector method, and the particle gun method.

The particle gun method is known to be one of the most useful methods for introducing exogenous genes into cultured cells or fertilized eggs. In the past, the particle gun was mainly used to introduce exogenous genes into plant cells (having cell walls) to produce many new plant varieties. However, in recent years, it has been suggested that the particle gun method should also be used to introduce exogenous genes into animal eggs to produce new animal varieties.

In fact, the particle gun method may be used to introduce exogenous genes into sea urchin eggs, or to introduce exogenous genes into eggs of some other invertebrates. Further, with some improvements, the particle gun method can be used to introduce exogenous genes into fish eggs covered with relatively hard shells, making it possible to treat simultaneously a large amount of fish eggs so as purposefully and effectively to produce new varieties of fishery products.

When the particle gun method is used to introduce exogenous genes into animal eggs, gold or tungsten particles coated with gene (DNA) material are accelerated by means of a pressurized gas so as to be injected into cultured cells or fertilized eggs. After introduction of exogenous genes into animal eggs in this way, the genes introduced in the eggs may be found on prism (late) embryos, whilst the metal particles can be removed from embryos at a later stage.

However, when any of the above prior art methods is used to introduce exogenous genes into cultured cells or fertilized eggs, there is a common problem: it is impossible to control whereabouts in the chromosome DNA the exogenous genes are introduced. As a result the introduced exogenous genes are generally exposed to an environment in which they may be influenced by adjacent genes in the cultured cell or fertilized egg.

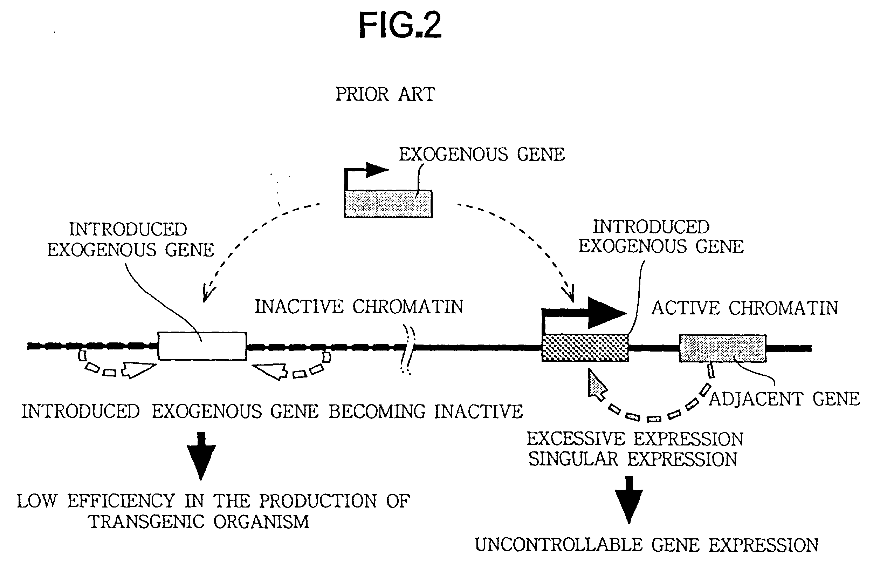

As indicated in Fig.2, when an exogenous gene is introduced close to inactive chromatin DNA, the introduced exogenous gene will become inactive due to the influence of the inactive chromatin DNA. As a result, there will be only extremely low efficiency in the production of transgenic organisms. On the other hand, when an exogenous gene is introduced close to active chromatin DNA, the introduced exogenous gene will become active due to the influence of the active chromatin DNA. This is because the introduced gene will be affected by strong enhancers in the active chromatin DNA, resulting in problems such as excessive expression of the introduced genes which have become period-singular or organization-singular, making it impossible to produce the desired transgenic organism.

### SUMMARY OF THE INVENTION

It is an object of the present invention to provide an improved method of exogenous gene introduction, permitting correct expression of introduced exogenous genes so as to produce desired transgenic organisms, thereby solving the above-mentioned problems peculiar to the above-mentioned prior art. Further objects include new materials useful in practising the method of the invention

According to the present invention, there is provided a method of introducing an exogenous gene into cultured cells or fertilized eggs, characterized in that the exogenous gene is introduced into chromosome DNA in a cultured cell or fertilized egg between insulators so as to insure an insulated environment, such that the introduced exogenous gene is shielded from influence from adjacent genes in the chromosome DNA.

Insulator elements are known to the art. They are defined by two characteristic effects on gene expression: they confer position-independent transcription on transgenes stably integrated in the chromosome (in particular protecting from the influence of adjacent chromatin) and they buffer a promoter from activation by an enhancer when located between the two (see David A. Gdula *et al*, Proc. Natl. Acad. Sci. USA 93, 9378-9383, 1996: "Genetic and molecular analysis of the gypsy chromatin insulator of *Drosophila*"). Several insulators are mentioned in the literature: they vary in length from a few hundred to a few thousand basepairs, and seem to lack common structural features. They are also known as domain boundaries. Tests have been described in the literature by which the efficiency of candidate insulators can be assessed: Jay H Chung *et al*, in Cell 74, 505-514, 1993: "A 5' element of the chicken β-globin domain serves as an insulator in human erythroid cells and protects against position effects in *Drosophila*", describe a test useful for assessing the efficiency of an insulator in inhibiting interference from adjacent chromatin; while Kellum and Schedl (Mol. Cell. Biol. 12, 2424-2431, 1992 "A group of scs elements function as domain boundaries in an enhancer-blocking assay") describe a test that can be used to measure efficiency of an insulator in blocking the effect of an enhancer.

According to one aspect of the present invention, the insulators are insulator fragments obtained from an upstream region of the urchin arylsulfatase gene. In detail, the insulators are insulator fragments which before use exist in a range of -2686∼-2115bp in an upstream region of the urchin arylsulfatase gene. The sequence of the urchin arylsulfatase gene and associated upstream region has been published by the present inventors in Dev. Growth & Differ. 36, 633-636, 1994, Akasaka *et al*, Our most recent work suggests that the main activity of the insulator function resides in the 184 basepair sequence in the upstream region of the urchin arylsulfatase gene from -2298bp to -2115bp from the start of transcription.

According to another aspect of the present invention, the insulator fragments are combined with plasmids of exogenous genes, such that each exogenous gene to be introduced into a cultured cell or fertilized egg is placed between insulator fragments.

According to a further aspect of the present invention, the insulator fragments are capable of completely shutting off the activity of enhancers of an arylsulfatase gene upon being inserted between enhancers and promoters of the arylsulfatase gene.

The invention further comprises a recombinant DNA sequence useful in the new process which comprises a functional gene including a promoter and coding sequence flanked by a pair of insulators which serve to shield the gene from the influence of adjacent DNA beyond each insulator. By the term 'functional gene' we mean DNA capable of generating RNA by transcription in the organism into which it is introduced. Such RNA may be mRNA capable of being transcribed into protein, or other RNA affecting the biochemistry of the organism, for example antisense RNA.

In a further aspect, the invention comprises a recombinant DNA sequence useful in introducing exogenous genes into cultured cells or fertilized eggs, comprising a functional gene including a promoter and coding sequence flanked by an insulator which serves to shield the gene from the influence of adjacent DNA beyond the insulator, the insulator comprising an insulator fragment derived from an upstream region of the urchin arylsulfatase gene in a range of -2686bp∼-2115bp from the start of transcription.

The above objects and features of the present invention will be more fully understood from the following description with reference to the accompanying drawings.

### BRIEF DESCRIPTION OF DRAWINGS

Figure 1 is a schematic explanatory view indicating the mechanism of a method according to the present invention.

Figure 2 is a schematic explanatory view indicating the mechanism of a method according to the prior art.

### DETAILED DESCRIPTION OF THE PREFERRED EMBODIMENTS

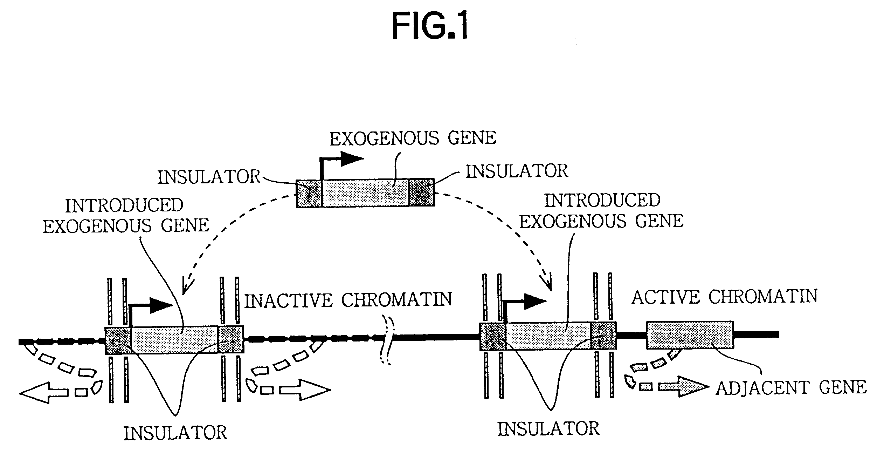

Referring to Fig. 1, in a method according to the present invention, an exogenous gene is introduced into chromosome DNA in a cultured cell or fertilized egg, the exogenous gene being placed between insulators so as to ensure an insulated environment, such that the introduced exogenous gene will be shielded from influence from adjacent genes in the cultured cell or fertilized egg.

It is understood from Fig. 1 that when an exogenous gene is introduced in a position close to inactive chromatin DNA, the introduced exogenous gene, owing to the effects of insulators positioned on both sides thereof, will not become inactive since the influence from the inactive chromatin DNA will be shut off by the insulators. On the other hand, when an exogenous gene is introduced in a position close to active chromatin DNA, the introduced exogenous gene, also owing to the effects of insulators positioned on both sides thereof, will not become too active since the insulators are able to shut off the effect of strong enhancers in the active chromatin DNA, thereby avoiding problems such as excessive expression of the introduced exogenous gene, or expression of an introduced exogenous gene which has become period-singular or organization-singular.

In this way, since it is possible to shield the influence from adjacent genes, each exogenous gene introduced into a cultured cell or fertilized egg is much more likely to express normally and stably on the chromosome DNA thereof, irrespective of (without having to depend on) the insertion position of the exogenous gene in the chromosome DNA, making it possible to produce desired transgenic organisms with improved efficiency.

The methods and materials of the present invention will be described in more detail below with reference to the following example.

The insulators used in the following example are insulator fragments which before use exist in a range of - 2686bp ∼-2115bp in an upstream region of the urchin arylsulfatase gene, reckoning from the start of transcription as origin.

The insulator fragments are combined with plasmids of exogenous genes, such that each exogenous gene to be introduced into a cultured cell or fertilized egg is placed between insulator fragments. In this way, the introduced exogenous gene will be shielded from adjacent genes in the cultured cell or fertilized egg.

During the actual operation for introducing genes into cultured cells of fertilized eggs, exogenous genes (DNA) and the insulator fragments are all linearized with a restriction enzyme, so that they all possess the same restriction enzyme ends. Any suitable restriction enzyme that does not cleave any functional region of the DNA may be used: in the example described below the restriction enzymes used were BamH1 or Sac1. By mixing the exogenous genes (DNA) with insulator fragments and then introducing the exogenous genes thus treated into cultured cells or fertilized eggs, the insulator fragments can provide the above-mentioned insulating effect.

### EXAMPLE

### Preparation of Fertilized Eggs

Sea urchin eggs obtained from the sea around Japan were fertilized in seawater. Then, the fertilized urchin eggs were washed twice using the same seawater. Afterwards, approximately 0.15 ml of the fertilized eggs were placed on a filter paper moistened with seawater. In this way, the fertilized eggs were caught up on fibers of the filter paper so as to be fixed on the filter paper.

### Preparation of DNA-coated Gold Particles

DNA containing a desired gene to be introduced into fertilized eggs was linearized with the use of a restriction enzyme to obtain restriction enzyme ends. Then, the DNA thus treated was mixed with urchin chromosome DNA (completely digested with the use of the same restriction enzyme and in amount 8 times that of the above treated gene), and also mixed with insulator fragments which had been linearized to obtain the same restriction enzyme ends, thus producing a DNA solution having a DNA concentration of 0.5 mg/ml.

Afterwards, 2.5 mg of dry gold particles (having a diameter of 1 µ and having been soundwave-washed in 100% ethanol solution) were suspended in 20 µl of the above DNA solution. Further added into the above DNA solution was 12 µl of autoclave-disinfected PEG solution (which may be obtained by dissolving polyethylene glycol in 2.5 M NaCl so that the solution has a PEG concentration of 20% by weight), followed by ice-cooling for 20 minutes. In this process, the DNA was deposited on the surfaces of the gold particles. Subsequently, the upper layer of the DNA solution was removed by virtue of centrifugal separation, thus obtaining DNA-coated gold particles. Finally, the DNA-coated gold particles were subjected to a further treatment by being suspended in 62.5 µl of 100% ethanol in a mixer.

### Introduction of DNA containing an Exogenous Gene into Fertilized Eggs

0.8 mg of DNA-coated gold particles were placed on the surface of a polyethylene projectile and were loaded into a particle gun, so that the DNA-coated particles could be projected outwardly with an initial speed of 350 m/s under a pressure of 0.1 atmosphere, thereby obtaining sufficient energy for the DNA-coated particles to bombard and penetrate the fertilized eggs fixed on the above filter paper.

### Evaluation of Expressibility of Exogenous Genes Introduced into Fertilized Eggs

Reporter genes such as CAT (chloramphenicol acetyltransferase) and Luc (luciferase) were used to evaluate the expressibility of the exogenous genes introduced into fertilized eggs. This confirmed that, using insulators as described above, introduced exogenous genes are not affected by influence from adjacent genes in the cultured cells or fertilized eggs.

Here, the CAT and Luc genes were used as experimental genes which were respectively combined at downstream sides of promoters of the histon H1 gene and the urchin arylsulfatase gene. By measuring the activities of these gene fusion products, one can confirm whether the introduced exogenous genes have expressed normally (in a desired manner) or not.

In detail, Luc was combined at the downstream side of the promoter area of the arylsulfatase gene to obtain a product Ars 100-Luc, while CAT was combined at the downstream side of the promoter area of the histon H1 gene to obtain a product H1-CAT. Then, Ars 100-Luc and H1-CAT were linearized with the use of the same restriction enzyme, and were introduced into fertilized eggs. In this way, the introduced Ars 100-Luc and H1-CAT were fused together and combined with the exogenous genes previously introduced in fertilized eggs. As a result, it was found that arylsulfatase gene had expressed 6 times as much as normal, thereby confirming that the previously introduced exogenous genes had expressed abnormally.

When the Ars 100-Luc construct was ligated with the arylsulfatase insulator fragments at both ends in accordance with the invention, in the same experimental conditions described above, abnormal expression was not observed, thereby confirming that the introduced exogenous genes had expressed normally (in a desired manner) when protected by insulator fragments.

It will be understood from the above description that, with the use of the method according to the present invention, when exogenous genes are introduced into the cultured cells or fertilized eggs, it is possible to shut off the influence from adjacent genes, so that exogenous genes introduced into the cultured cells or fertilized eggs are more likely to express normally (in a desired manner) and stably on chromosome DNA thereof, irrespective of (without having to depend on) insertion position of the exogenous gene in the chromosome DNA, making it possible to produce desired transgenic organisms with higher efficiency.
While the presently preferred embodiments of the invention have been shown and described above, it is to be understood that these disclosures are for the purpose of illustration and that various changes and modifications may be made without departing from the scope of the invention as set forth in the appended claims.

## Claims

1. A method of introducing an exogenous gene into cultured cells or fertilized eggs, characterized in that the exogenous gene is introduced into chromosome DNA in a cultured cell or fertilized egg between insulators so as to insure an insulated environment, such that the introduced exogenous gene is shielded from influence from adjacent genes in the chromosome DNA.

2. A method as claimed in claim 1, wherein the insulators are insulator fragments obtained from an upstream region of the urchin arylsulfatase gene.

3. A method as claimed in 2, wherein the insulators are insulator fragments derived from an upstream region of the urchin arylsulfatase gene in a range of - 2686bp∼-2115bp from the start of transcription.

4. A method as claimed in 3, wherein the insulator fragments comprise the 184 basepair sequence in the upstream region of the urchin arylsulfatase gene from -2298bp to -2115bp from the start of transcription.

5. A method as claimed in any of claims 1-4, wherein the insulator fragments are combined in a plasmid with the exogenous gene, such that the exogenous gene to be introduced into a cultured cell or fertilized egg is placed between insulator fragments.

6. A recombinant DNA sequence useful in the method claimed in any of claims 1-5 which comprises a functional gene including a promoter and coding sequence flanked by a pair of insulators which serve to shield the gene from the influence of adjacent DNA beyond each insulator.

7. A recombinant DNA sequence as claimed in claim 6 in which at least one of the insulators comprises an insulator fragment derived from an upstream region of the urchin arylsulfatase gene in a range of -2686bp∼-2115bp from the start of transcription.

8. A recombinant DNA sequence useful in the process claimed in any of claims 1-5 comprising a functional gene including a promoter and coding sequence flanked by an insulator which serves to shield the gene from the influence of adjacent DNA beyond the insulator, the insulator comprising an insulator fragment derived from an upstream region of the urchin arylsulfatase gene in a range of - 2686bp∼-2115bp from the start of transcription.

9. A recombinant DNA sequence as claimed in claim 8 in which the insulator fragment comprises the 184 basepair sequence in the upstream region of the urchin arylsulfatase gene from -2298bp to -2115bp from the start of transcription.

10. A plasmid useful for transforming eukaryotic organisms which comprises recombinant DNA as claimed in any of claims 6-9.

11. A recombinant organism transformed with DNA claimed in any of claims 6-9.
